Europäisches Patentamt

European Patent Office

Office européen des brevets

(11) Publication number: 0 520 604 A2

## EUROPEAN PATENT APPLICATION

(21) Application number: 92301763.6

(22) Date of filing: 02.03.92

(51) Int. Cl.5: **C12P 21/08**, C12N 5/20,
C12N 15/06, G01N 33/577,
G01N 33/94

The microorganism(s) has (have) been deposited with Fermentation Research Institute under number(s) FERM BP-3389.

(30) Priority: 25.06.91 JP 180334/91

(43) Date of publication of application:
30.12.92 Bulletin 92/53

(84) Designated Contracting States:
**DE FR GB IT**

(71) Applicant: **MINOPHAGEN PHARMACEUTICAL COMPANY**
**2-7, 3-chome, Yotsuya Shinjuku-ku Tokyo(JP)**

(72) Inventor: **Mizugaki, Michinao**
**7-33, Kunimi 1-chome, Aoba-ku**
**Sendai-shi, Miyagi-ken(JP)**
Inventor: **Ishida, Nakao**
**5-40, Tsunogoro 1-chome, Aoba-ku**
**Sendai-shi, Miyagi-ken(JP)**
Inventor: **Itoh, Kunihiko**
**15-5, Izumigaoka 5-chome, Izumi-ku**
**Sendai-shi, Miyagi-ken(JP)**
Inventor: **Takeuchi, Tadao**
**4-17 Fukuda 7-chome**
**Yamato-shi, Kanagawa-ken(JP)**

(74) Representative: **Myerscough, Philip Boyd**
**J.A. Kemp & Co. 14 South Square, Gray's Inn**
**London WC1R 5LX(GB)**

(54) Monoclonal antibody against pentacyclic oleanan-type triterpene and use thereof.

(57) The invention relates to a monoclonal antibody capable of recognising pentacyclic oleanan-type triterpene, use of said antibody as a diagnostic reagent, a kit containing said antibody and a method for the production of said antibody.

EP 0 520 604 A2

## BACKGROUND OF THE INVENTION

The present invention relates to a monoclonal antibody capable of recognizing oleanan-type five-membered triterpene and a hybridoma generating the monoclonal antibody.

Glycyrrhetic acid is the oleanan-type five-membered triterpene constituting glycyrrhizin as one of the effective components of licorice. It is known that glycyrrhizin is metabolized in biological bodies into glycyrrhetic acid.

Glycyrrhizin is a pharmaceutical agent widely used for the therapeutic treatment of patients with liver diseases. If it is continuously administered at a higher dose, adverse effects such as pseudo-aldosteronism and hypokalemia might possibly be triggered. Thus, it has been expected accurate and highly sensitive assay of the concentration of glycyrrhetic acid in blood.

High-performance liquid chromatography (HPLC), gas chromatography, etc. have now been used for the assay of trace components, but the measurement according to these methods requires complex preliminary procedure. Furthermore, qualitative findings including precise retention time, etc. are demanded for the mesurement. Alternatively, immunoassay using antibodies has been developed and applied to the assay of trace components; furthermore, enzyme immunoassay with no use of radioactive substances for measuring the amount of an antibody bonded to an antigen. Thus, extremely accurate and highly sensitive assay has been realized without using specific laboratory facilities.

In order to carry out immunoassay, an antibody to a subjective substance is required. Since a lower-molecular hapten per se, such as glycyrrhetic acid, has no antigenicity, antibodies are generated by immunizing animals with those haptens bonded with carrier proteins. Kanaoka et al. report the method of producing an antibody to glycyrrhetic acid, comprising immunizing rabbits with glycyrrhetic acid bonded with carrier protein thereby producing antiserum, and subsequently purifying the antiserum to produce an antibody (PHARM TECH JAPAN, Vol. 4, 913-920, 1988).

However, it is observed that polyclonal antibodies have some cross reactivity to other biological components, and that such antibodies have a defect in that the amount of antibodies to be obtained is limited. Hence, the supply of monoclonal antibodies have been demanded.

In order to satisfy the above demand, the present invention is to provide a monoclonal antibody capable of recognizing an oleanan-type five membered triterpene, and a method of measurement, a diagnostic kit and a screening method using the same.

## SUMMARY OF THE INVENTION

The present invention is intended to solve the above problems, by producing a monoclonal antibody capable of recognizing oleanan-type five-membered triterpene by means of a hybridoma obtained by fusing a spleen cell obtained by using as immunogen the oleanan-type five membered triterpene bonded to carrier protein to a myeloma cell, and using the monoclonal antibody for the method to measure the oleanan-type five-membered triterpene in a sample according to immunological methods.

The present invention will now be explained in the steps of preparing a monoclonal antibody hereinafter.

1. Bonding of an oleanan-type five-membered triterpene to carrier protein.

Since a lower-molecular hapten such as oleanan-type five-membered triterpene per se has no antigenicity, antibodies to the oleanan-type five-membered triterpene, namely the antibodies recognizing and bonding the oleanan-type five membered triterpene, can be generated by using such hapten bonded with carrier protein, as the immunogen.

Explanation will follow by illustrating glycyrrhetic acid as one such oleanan-type five-membered triterpene.

It is required to constitute a cross-liking part in order to bond glycyrrhetic acid to carrier protein, and as such cross-linking part, glycine, γ-aminobutylic acid, ε-aminohexanoic acid, etc. can be used as the cross-linking part. Glycine is particularly preferable.

In order to bond glycine to glycyrrhetic acid, the procedure hereinbelow should be followed. Using a condensing agent such as dicyclohexylcarbodiimide (DDC), diphenylphosphonyl azide (DPPA) or diethylphosphoryl cyanide (DEPC) and the like in an organic solvent, glycine methylester is coupled to glycyrrhetic acid to obtain glycyrrhetinylglycine methylester. The subsequent hydrolysis thereof produces glycyrrhetinylglycine.

The bonding of the glycyrrhetic acid bonded to glycine (referred to as GA-Gly hereinafter) with a carrier can be effected by introducing an activated group into GA-Gly by a condensing agent, and reacting the activated GA-Gly with carrier protein. As to the condensing agent, a compound having a carbodiimide configuration is preferable, and water-soluble carbodiimide [1-ethyl-3-(3-dimethylaminopropyl)-carbodiimide] is specifically preferable. Amide ester is preferable as the activated group, and succinimide group is specifically preferable.

Highly immunogenic proteins can be used as the carrier protein and key-hole limpet hemocyanin (KLH), bovine serum albumin (BSA) or ovalbumin (OVA) is preferable.

The method to bond glycyrrhetic acid to carrier protein is explained in a concrete manner.

The bonding of GA-Gly to carrier protein (KLH or BSA) is effected by N-hydroxysuccinimide procedure. The carboxyl group of GA-Gly is activated with water-soluble carboiimide [1-ethyl-3-(3-dimethylaminopropyl)-carbodiimide] to be introduced into the N-hydroxysuccinimide activated ester of GA-Gly (referred to as GA-Gly-NHS hereinbelow). By reacting a KLH- or BSA solution with the activated ester, KLH- or BSA conjugated GA-Gly can be obtained.

More specifically, GA-Gly is dissolved in 80 % dioxane, followed by the addition of N-hydroxysuccinimide and water-soluble carbodiimide for reaction at room temperature for 2 hours. Subsequent extraction using ethyl acetate generates the succinimide activated ester of GA-Gly (GA-Gly-NHS).

After dissolved in a small amount of dimethylsulfoxide, the GA-Gly-NHS was added to a KLH- or BSA solution and reacted at 4° C for 24 hours. By dialyzing further the reaction solution in 10mM phosphate buffered physiological solution (pH 7.4; referred to as PBS hereinafter ) followed by freeze drying, there can be generated a conjugate of GA-Gly and KLH (referred to as GA-KLH conjugate), or a conjugate of GA-Gly and bovine serum albumin (BSA, referred to as GA-BSA conjugate hereinbelow).

The generation of succinimide activated ester can be confirmed on thin-layer chromatography (referred to as TLC hereinbelow). Alternatively, the generation of the conjugates can be confirmed by the shift of the peak in the UV absorption spectra of carrier protein at 280 nm toward 252 nm derived from glycyrrhetic acid.

## 2. Immunization of mouse by GA-carrier protein conjugate

A solution of the GA-carrier protein conjugate is mixed with an equal amount of Freund complete adjuvant. The resulting mixture is thoroughly emulsified, which is then injected subcutaneously or intraperitoneally into a BALB/c mouse. Subsequently, a mixed emulsion of a GA-carrier protein conjugate solution with an equal amount of Freund complete adjuvant is injected intraperiotoneally twice for booster. Such booster generally may be carried out at an interval of two weeks.

Ten days after the second booster, the GA-carried protein conjugate solution is intravenously injected.

The concentration of the GA-carrier protein conjugate to be used is near 200 to 400 $\mu$g/m$\ell$. Satisfactorily, the subcutaneous or intraperitoneal dose may be about 0.5 m$\ell$; the intravenous dose, about 0.2 m$\ell$.

Before final immunization, it is preferable to confirm the increase of the antibody titer in blood, according to enzyme immunoassay.

## 3. Preparation of hybridoma

Three days after final immunization, spleen is resected aseptically from the mouse to be used for the cell fusion thereof with a myeloma cell derived from a mouse of the same strain. The cell fusion is specifically carried out as follows.

Myeloma cells frozen under storage are washed and suspended in a medium, followed by the culture in a culturing flask to obtain cells during a logarithmic growth phase. As the medium, there may be used the RPMI 1640 medium to which was added fetal calf serum at a proportion of 10 % (referred to as FCS-RPMI hereinbelow). As to RPMI 1640 medium, see Moore, G.E., et al., J.A.M.A. 199; 519 (1967), etc.

Spleen cells and myeloma cells are collected separately with centrifuge, followed by washing several times in a medium, and they are mixed well. On adding 0.5 m$\ell$ of 50 % polyethylene glycol 4000, cell fusion is effected. The fused cells are washed in the medium, before resuspension of the cells into the FCS-RPMI medium, and divided into a 96-well microtiter plate for the culture in a carbonate gas incubator.

The fused cells are selected according to HAT selection. The method of the HAT selection is described in Science, Vol. 145, page 709, 1964, and the principle of the selection is as follows.

The myeloma to be used for the cell fusion is deficient in hypoxanthine-guanine-phosphoribosyl transferance (HGPRT), so nucleic-acid synthesis is dependent on de novo synthetic pathway. Even if the de novo synthetic pathway is inhibited by aminopterin, the fused cells of such myeloma cells with normal lymphocytes can perform nucleic-acid synthesis in HAT medium through the salvage pathway derived from the lymphocytes, because of the presence of thymidine and hypoxanthine. Therefore, the fused cells can grow in HAT medium (containing hypoxanthine, aminopterin and thymidine). Then, the myeloma cells are eliminated because the de novo synthetic pathway is inhibited by aminopterin so that the cells cannot synthesize nucleic acids. Alternatively, it is impossible to culture lymphocytes for a prolonged period because they are normal cells. Consequently, only the hybridomas generated by the fusion of myeloma cells and lymphocytes can grow in HAT medium, so fused cells can be selected from non-fused cells.

The potency of a hybridoma to generate antibodies and the antibody titer thereof, can be examined according to enzyme immunoassay shown hereinbelow.

4. Enzyme immunoassay (Direct ELISA, Inhibition ELISA)

The potency of a hybridoma to generate antibodies and the antibody titer thereof are assayed according to direct ELISA using the GA-BSA conjugate as antigen; and the cross reactivity of the antibodies and the amount of the antigens in various samples are determined according to inhibition ELISA using glycyrrhetic acid as a standard substance.

Direct ELISA comprises sequentially incubating the antigen namely solidified GA-BSA conjugate, with an antibody solution, a solution of an enzyme-labeled anti-mouse immunoglobulin G (anti IgG) prepared in animals except mouse, and measuring the activity of the enzyme bonded through the antibody to the antigen, thereby assaying the antibody.

Inhibition ELISA comprises sequentially incubating the solidified GA-BSA conjugate with glycyrrhetic acid or a sample together with the antibody, bonding the enzyme-labeled anti-mouse IgG to the resulting incubation mixture, thereby measuring the enzyme activity. The antibody bonded to the antigen contained in the sample cannot be bonded to the solidified antigen, and therefore, the amount of the enzyme bonded is reduced. By measuring the enzyme activity, glycyrrhetic acid contained in the sample can be measured. The amount of glycyrrhizin can be determined by measuring glycyrrhetic acid generated by the acid hydrolysis of glycyrrhizin.

For the solidification of antigens, agarose, latex particles, microtiter-plate wells and the like, can be used, and microtiter-plate wells are more preferable.

One example of ELISA thereof is now shown.

An antigen solution (for example, GA-BSA) is divided into the wells of a 96-well microtiter plate, and left to stand overnight at 4° C. After removing the antigen solution, a blocking solution for example PBS containing 1 % BSA, is divided therein and left to stand 37° C, for effecting blocking.

According to direct ELISA, the antibody solution is divided into each well; according to inhibition ELISA, glycyrrhetic acid or a sample solution is divided into each well together with the antibody solution. After left to stand at ambient temperature, the wells are washed before the division of an enzyme-labeled anti-mouse IgG solution, and then the wells are left to stand at ambient temperature.

The enzyme for labeling is preferably alkaline phosphataseand goat anti-mouse IgG labeled with alkaline phosphatase can be commercially available from Tago, Ltd.

After washing each well again, a solution of a dye which develops color on enzyme reaction is divided into the washed wells for reaction. After the completion of the reaction, the absorbance of each well at 405 nm is measured with an immunoreader. By comparing the results with the values obtained from known amounts of the antigen, glycyrrhetic acid can be assayed.

As to the dye, p-nitrophenyl phosphate is used when anti-mouse IgG labeled with alkaline phosphatase is used.

The enzyme to label anti-mouse IgG may be horseradish peroxidase. In such case, ABTS [2,2'-adino-di-(3-ethylbenzothyazolin-sulfonate)], TMBZ (tetramethyl benzidine), OPD (O-phenylene diamine) and the like can be used as the substrate, instead of p-nitrophenyl phosphate.

Glycyrrhetic acid or glycyrrhizin can be measured according to the methods described above. A simple and rapid test can be done by making reagents necessary for the assay including the monoclonal antibody, a secondary antibody labeled with enzyme and the like into a kit.

One example of such kit is illustrated as the one comprising a microtiter plate, BSA, GA-BSA, monoclonal antibody, washing buffer solutions, alkaline phosphatase-labeled goat anti-mouse IgG, p-nitrophenyl phosphate, glycyrrhtic acid of known levels for standards for the assay. Preferably, a microtiter plate is prelimiarily solidified with GA-BSA and blocked with BSA.

Such kit is preferably shipped, after antibodies, BSA, GA-BSA or dyes are prepared into freeze-dry products or they are dissolved into solvents capable of stably storing them. In case that such kit is shipped in solution, it is possible to add a bactericidal agent and the like to the kit, but such agent is preferably the one which does not inhibit enzyme reaction.

5. Application of the monoclonal antigen according to the present invention to imaging diagnostics.

The monoclonal antibody recognizing oleanan-type five-membered triterpene in accordance with the present invention can be used for imaging diagnostics after the labeling thereof with radioactive substances, para-magnetic substances, fluorescent substances or the like.

The labeling of the monoclonal antibody with a radionuclide $^{125}$I can be effected according to chloramine-T method. For para-magnetic substances and fluorescent substances, methods comprising bonding such substances to monoclonal-antibody molecules using heterobifunctional cross-linking agents such as SPDP, GMBS and the like are illustrated.

According to the present invention, the monoclonal antibody specific to oleanane-type five-membered triterpene can be prepared. Using the present antibody, glycyrrhizin and glycyrrhetic acid can be assayed specifically with a high sensitivity.

BRIEF DESCRIPTION OF THE DRAWINGS

Fig. 1 is a graph of the UV absorption spectra of GA-KLH conjugate;
Fig. 2 is a graph of the UV absorption spectra of GA-BSA conjugate;
Fig. 3 represents graphs of the titer of the monoclonal antibody produced by a hybridoma strain GA2-20-1, wherein the ordinate represents absorbance and the abscissa represents dilution factor (Figs. 4 and 5 are represented in the same manner);
Fig. 4 represents graphs of the titer of the monoclonal antibody produced by a strain GA2-20-3;
Fig. 5 represents graphs of the titer of the monoclonal antibody produced by a strain GA2-20-6;
Fig. 6 represents graphs of the cross reactivity between the monoclonal antibody produced by a strain GA2-20-1 and a compound (1), (2), (3), (4), (GA) or (GL), wherein the ordinate represents inhibition ratio and the abscissa represents the concentration of an inhivitive agent (Figs. 7, 8 and 9 are shown in the same manner);
Fig. 7 represents graphs of the cross reactivity between the monoclonal antibody produced by a strain GA2-20-1 and a compound (5) or (6); Fig. 8 represents graphs of the cross reactivity between the monoclonal antibody produced by a strain GA2-20-3 and a compound (1), (2), (3), (4), (GA) or (GL);
Fig. 9 represents graphs of the cross reactivity between the monoclonal antibody produced by a strain GA2-20-3 and a compound (5) or (6);

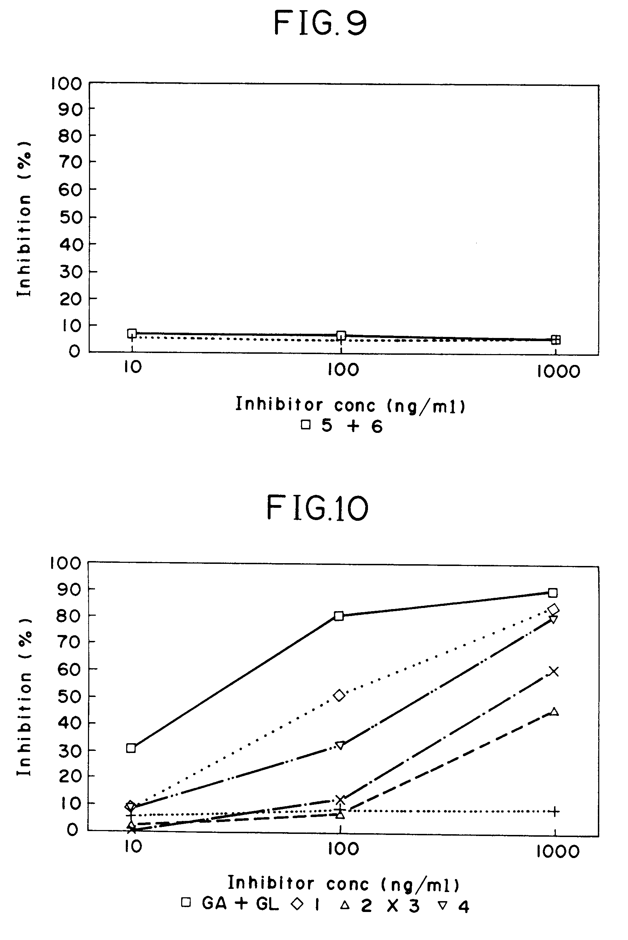

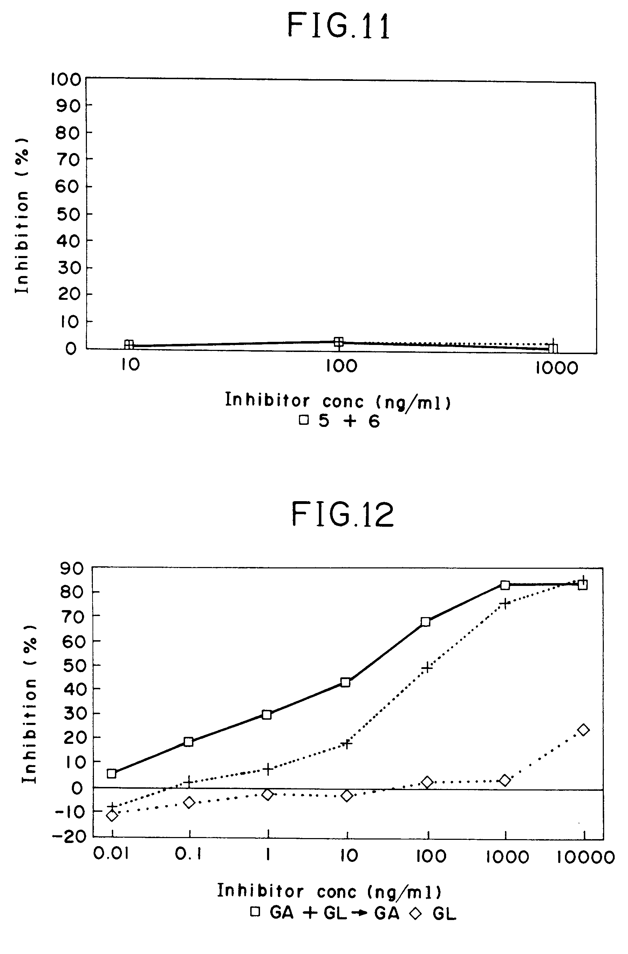

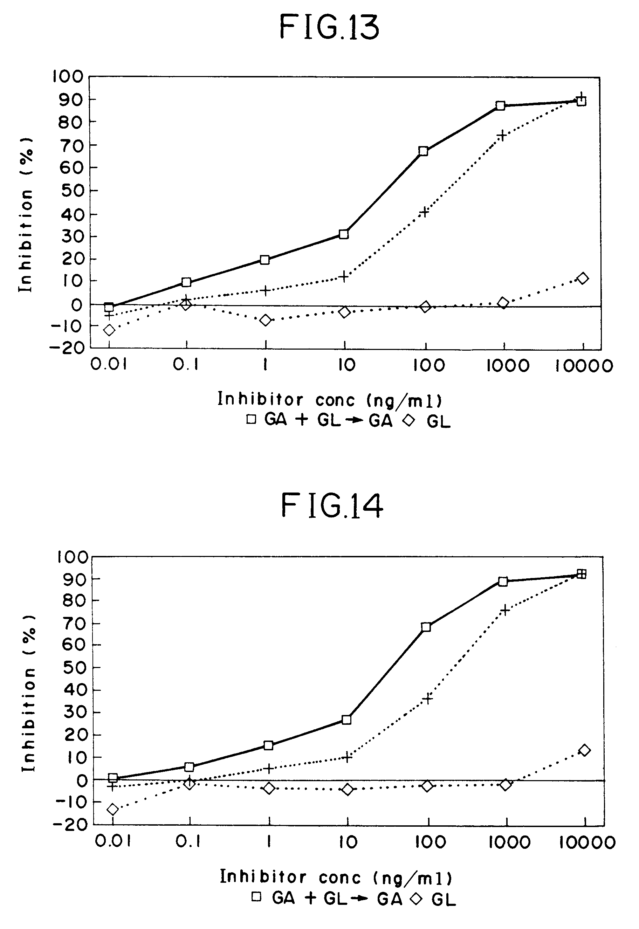

Fig. 10 represents graphs of the cross reactivity between the monoclonal antibody produced by a strain GA2-20-6 and a compound (1), (2), (3), (4), (GA) or (GL); Fig. 11 represents graphs of the cross reactivity between the monoclonal antibody produced by a strain GA2-20-6 and a compound (5) or (6); Fig. 12 represents graphs of glycyrrhetic acid and glycyrrhizin, assayed with the monoclonal antibody produced by a strain GA2-20-1; Fig. 13 represents graphs of glycyrrhetic acid and glycyrrhizin, assayed with the monoclonal antibody produced by a strain GA2-20-3; Fig. 14 represents graphs of glycyrrhetic acid and glycyrrhizin, assayed with the monoclonal antibody produced by a strain GA2-20-6.

DESCRIPTION OF THE PREFERRED EMBODIMENTS

1. Binding of glycyrrhetic acid and carrier protein

GA-Gly (10 mg; 19 $\mu$mol) was dissolved in 0.2 mℓ of 80 % dioxane and 20 % H$_2$O, followed by the addition of 2.1 mg (18 $\mu$mol) of N-hydroxysuccinimide and 5.6 mg (29 $\mu$mol) of water-soluble carbodiimide, for the reaction at ambient temperature for 2 hours. The generation of the succinimide activated ester of GA-Gly (GA-Gly-NHS) was confirmed in the following fashion. A raw material, a reaction product and the mixture thereof were spotted on a sequentially layered silica gel thin layer plate, before the development with chloroform-methanol (3:1). By developing color using phosphorous molybdate, the reaction product was confirmed near the value of Rf 0.9.

Then, 8 mℓ of deionized water was added to the reaction solution, followed by the addition of 8 mℓ of ethyl acetate. Subsequently, the reaction product was extracted using a separatory funnel. After washing the ethyl acetate layer in deionized water three times, dehydration was effected by the addition of sodium sulfate, followed by the evaporation of the solvent, to produce the succinimide activated ester of GA-Gly (GA-Gly-NHS).

The GA-Gly-NHS obtained in the above-described manner was dissolved in 0.3 mℓ of dimethylsulfoxide, and then added to KLH solution or BSA solution (10 mg/2 mℓ PBS), for the reaction at 4° C for 24 hours. By dialyzing the reaction solution against PBS before freeze drying, GA-KLH conjugate or GA-BSA conjugate was obtained.

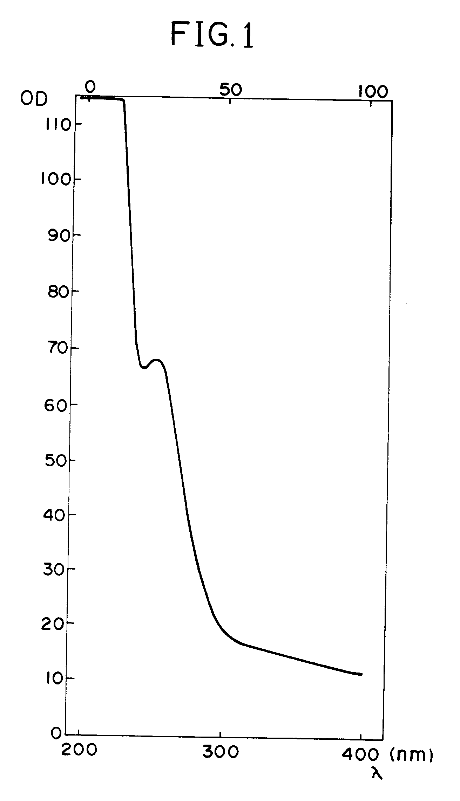

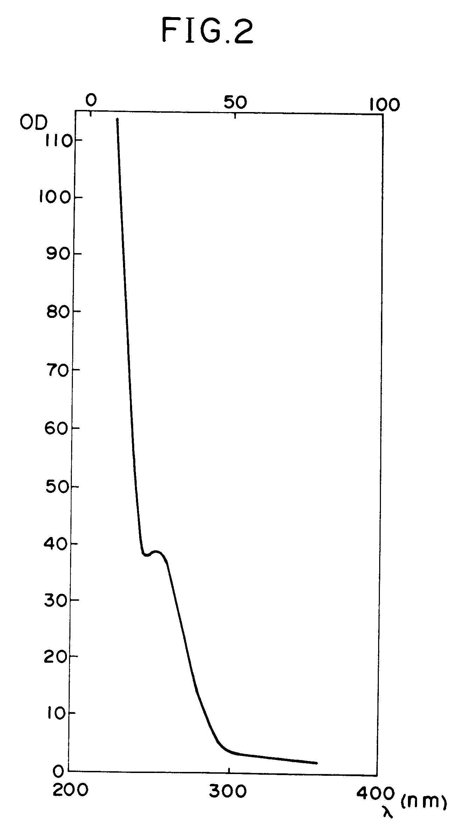

The generation of a conjugate was confirmed by the sift of the peak of the UV absorption spectra of the carrier protein at 280 nm toward 252 nm derived from glycyrrhetic acid (Figs. 1 and 2).

## 2. Immunization of mouse with GA-KLH conjugate

A solution of the GA-KLH conjugate (200 $\mu$g/m$\ell$ PBS) was mixed with an equal amount of Freund complete adjuvant and emulsified completely. The resulting thorough emulsion was injected at a dose of 0.5 m$\ell$ subcutaneously and intraperitoneally into a female BALB/c mouse aged 6 weeks. Thereafter at an interval of 14 days, the mixed emulsion of the GA-KLH conjugate solution (200 $\mu$g/m$\ell$ PBS) and an equal amount of Freund complete adjuvant was injected twice at 0.5 m$\ell$ each intraperitoneally. Seven days after a third injection, the increase of the antibody titer in blood was confirmed according to direct ELISA. Three days thereafter, 0.2 m$\ell$ of the GA-KLH conjugate solution (400 $\mu$g/m$\ell$) was intravenously boosted.

## 3. Preparation of hybridoma

Three days after the final immunization, spleen was aseptically resected from the mouse, which was then broken down and suspended in RPMI 1640 medium. The resulting suspension was used for the cell fusion with the myeloma cell line Sp2/0-Ag14-K13 from a mouse from the same strain. The myeloma cell line was supplied by Dr. Kotaro Tada of the Department of Bacteriology, School of Medicine, Tohoku University.

After washing the myeloma cell line Sp2/0-Ag14-K13 stored under freezing, the cells were suspended in FCS/RPMI ($5 \times 10^4$/m$\ell$), and cultured in a culturing flask to obtain cells during a logarithmic growth phase.

Spleen cells of $6 \times 10^7$ and myeloma cells of $1.25 \times 10^7$ were collected by centrifuge, washed three times in RPMI 1640 medium, to mix well the spleen cells and the myeloma cells, followed by the addition of 0.5 m$\ell$ of 50 % polyethylene glycol 4000 (average molecular weight of 3,000; Selling agency, Merck) for cell fusion.

The fused cells were washed once in RPMI 1640 medium, suspended in FCS-RPMI 1640 medium, and then divided in a 96-well microtiter plate at 0.1 m$\ell$ each for the culture in a carbonate-gas incubator.

## 4. Screening of antibody-generating hybridoma

Hybridomas were inoculated at $5 \times 10^4$/m$\ell$ into a culturing flask for the culture in a carbonate-gas incubator at 37° C for 3 to 4 days, and then the supernatant of the culture was recovered.

Each supernatant was assayed according to the direct ELISA using GA-BSA as the antigen and the inhibition ELISA using glycyrrhetic acid as a standard substance. More specifically, the procedure described below was similarly followed.

Consequently, the generation of specific antibodies to glycyrrhetic acid was observed in three strains of hybridomas. Thus, their cloning was effected by limiting dilution.

In a 96-well microtiter plate inoculated with $1 \times 10^6$/well of mouse thymus cells was applied 100 $\mu\ell$ each of a hybridoma-cell-suspended solution adjusted at 10 cells/m$\ell$ using FCS-RPMI medium, for the culture in a carobonate-gas incubator at 37° C. Seven to ten days later, hybridoma colonies were confirmed and wells with 1 colony formed per well were selected.

The three strains GA2-20-1, GA2-20-3 and GA2-20-6 were successfully established finally.

Among them, the GA2-20-6 was deposited as deposition number FERM BP-3389 to Agency of Industrial Science and Technology, Fermentation Research Institute.

Next, the titer of the monoclonal antibody generated by these strains was determined by direct ELISA.

One hundred $\mu\ell$ each of the GA-BSA solution (10 $\mu$g/m$\ell$ PBS) was divided in a polyvinylchloride 96-well microtiter plate (Sumitomo Bakelite, MS-7196F) and left to stand overnight at 4° C. After removing the antigen solution, 100 $\mu\ell$ each of the PBS containing 1 % BSA (BSA-PBS) was divided therein and left to stand at 37° C for one hour.

In order to avoid reaction between the antibody and carrier protein (KLH), the GA-BSA conjugate having no cross reactivity with KLH was used as the antigen.

The supernatant of the individual cultures of the hybridomas was sequentially 2-fold diluted to prepare a dilution series up to 1024 fold. One hundred $\mu\ell$ each of the dilution series was divided in each well. After left to stand at ambient temperature for 30 minutes, each well was washed three times in the PBS containing 0.05 % Tween 20 (polyoxyethylene sorbitan monolaurate) and washed twice in PBS, followed by the division of 100 $\mu\ell$ each of the 5000-fold dilution (PBS) of the goat anti-mouse IgG labeled with alkaline phosphatase (Tago, Ltd., 6542) and left to stand at ambient temperature for 30 minutes.

After washing each well in the same manner as in the aforementioned procedure, 100 $\mu\ell$ each of 1M diethanolamine buffer (pH 9.8) containing 1 mg/m$\ell$ of p-nitrophenyl phosphate was divided and reacted at 37° C for 30 minutes. After the completion of the reaction, 2M sodium hydroxide solution was added, and the absorbance at 405 nm of each well was measured with an immunoreader (manufactured by Bio-Rad, Ltd., Model 2550).

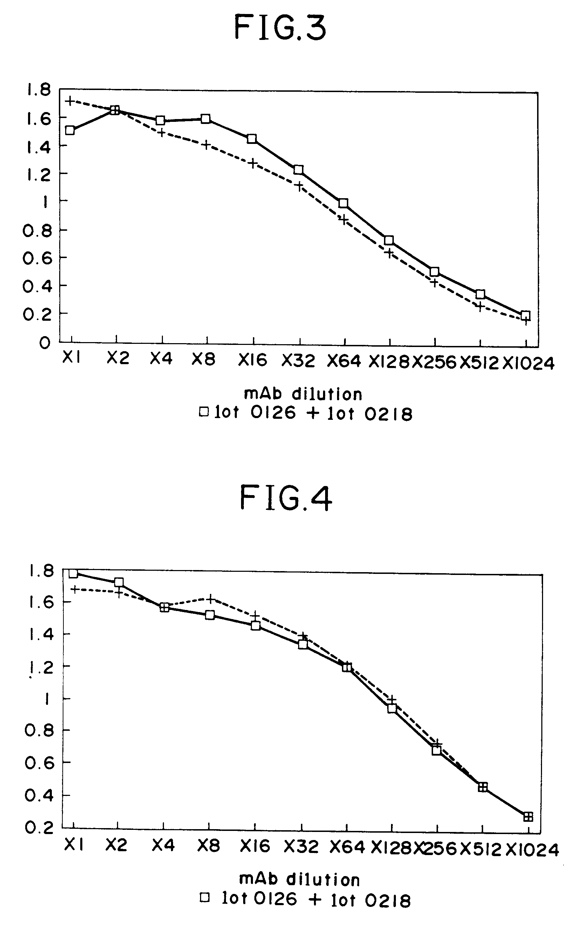

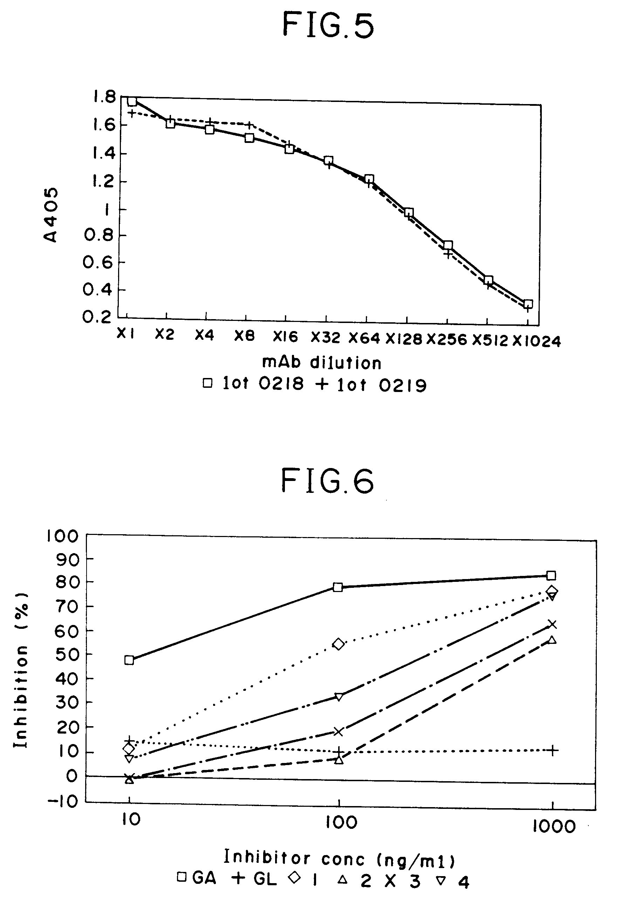

The results are shown in Figs. 4 and 5. provided that the dilution factor giving the absorbance of about 1.0 was regarded as the antibody titer, any antibody gave the absorbance of 1.0 at about 100-fold dilution. Therefore, such dilution factor was regarded as the antibody titer. The 50-fold diluted solution of each antibody was used in inhibition ELISA.

Using a mouse monoclonal antibody isotyping kit (manufactured by Amersham, RPN29), the class of these monoclonal antibodies were examined. It was found consequently that the class of any antibody was IgG1• $\varkappa$.

## 5. Examination of the cross reactivity of monoclonal antibody

The cross reactivity to the following individual compounds was examined according to inhibition ELISA.
(1) 18$\alpha$ H-Olean-11-oxo-12ene-30-oic acid
(2) Olean-12ene-3,11-dioxo-30-oic acid
(3) Olean-12ene-3$\alpha$-hydroxy-11-oxo-30-oic- acid
(4) Olean-12ene-11-oxo-3$\beta$, 30-diol
(5) Cholic acid
(6) Deoxy cholic acid
(7) Glycyrrhetlc acid (GA)
(8) Glycyrrhizin (GL)
These are illustrated as compounds (1), (2), (3), (4), (5), (6), (GA) and (GL).

One hundred $\mu\ell$ each of the GA-BSA solution (10 $\mu$g/m$\ell$) was divided in the wells of a 96-well microtiter plate and left to stand overnight at 4° C. After removing the antigen solution, 100 $\mu\ell$ each of the BSA-PBS was divided and left to stand at 37° C for one hour.

The 50-fold diluted antibody and each of the compounds were individually adjusted to 10 ng/m$\ell$, 100 ng/m$\ell$ and 1000 ng/m$\ell$. Fifty $\mu\ell$ each of the adjusted solution was divided in each well. After left to stand at ambient temperature for 30 minutes, the wells were washed three times with the PBS containing 0.05 % Tween 20, and washed twice with PBS, followed by the division of the 5000-fold dilution of goat anti-mouse IgG labeled with alkaline phosphatase, and left to stand at room temperature for 30 minutes.

After each well was washed following the same procedure described above, 100 $\mu\ell$ each of 1M diethanolamine buffer (pH 9.8) containing p-nitrophenyl phosphate at a concentration of 1 mg/m$\ell$ was divided and reacted at 37° C for 30 minutes. After the completion of the reaction, 2M sodium hydroxide was added. Then, the absorbance at 405 nm of each well was measured with an immunoreader.

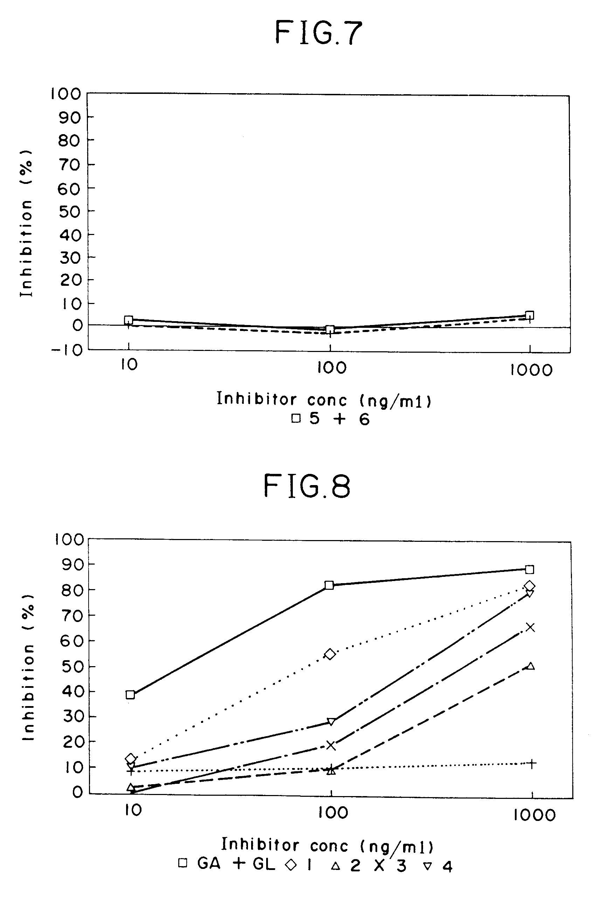

The results are shown in Figs. 6, 7, 8, 9, 10 and 11. From the inhibition curves obtained, 50 % inhibition concentration (IC 50) of each compound was determined. The antibody generated by the strain GA2-20-1, among the three strains, had the highest specificity.

Table 1 shows the IC50 values to the individual antibodies, wherein the IC50 values were obtained from the 50 % inhibitive concentrations obtained based on the inhibition curves prepared using each inhibitive agent.

Table 1

| compound | monoclonal antibody | | | | | |
|---|---|---|---|---|---|---|
| | 2-20-1 | | 2-20-3 | | 2-20-6 | |
| (1) | 87.6 | (18.2) | 87.8 | (37.5) | 97.5 | (45.5) |
| (2) | 831.7 | ( 1.9) | 957.1 | ( 3.4) | >1000 | (<1.6) |
| (3) | 695.4 | ( 2.3) | 676.5 | ( 4.9) | 793.3 | ( 5.6) |
| (4) | 437.2 | ( 3.6) | 476.7 | ( 6.9) | 425.4 | (10.4) |
| (5) | >1000 | (<1.6) | >1000 | (<1.6) | >1000 | (<1.6) |
| (6) | >1000 | (<1.6) | >1000 | (<1.6) | >1000 | (<1.6) |
| (GA) | 15.9 | | 32.9 | | 44.4 | |
| (GL) | >1000 | (<1.6) | >1000 | (<1.6) | >1000 | (<1.6) |
| Unit : ng/ml | | | | | | |
| Values in parenthesis indicate cross reactivity in %. | | | | | | |

From the results described above, it was indicated that the recognition site of the antibodies was in the environment including the structural configuration of glycyrrhetic acid, having the hydroxyl group at 3 position of the center thereof. From the results of the tests of the cross reactivity of individual steroidal compounds, it was confirmed that the antibodies did not react with any such compound.

6. Sensitivity to detect glycyrrhetic acid with monoclonal antibody, and detection of glycyrrhizin on the basis of the transformation thereof into glycyrrhetic acid

A solution of glycyrrhetic acid in ethanol (1 mg/mℓ) was diluted with PBS and prepared into the solution of 10 $\mu$g/mℓ. The solution was sequentially diluted 10 fold to prepare the solutions down to the concentration of 10 pg/mℓ. Inhibition ELISA was carried out following the same procedure described above, to examine the detection limit of glycyrrhetic acid.

Glycyrrhizin was detected according to inhibition ELISA, after the glycyrrhizin was transformed into glycyrrhetic acid by acid hydrolysis. More specifically, 200 $\mu$ℓ of 4N NC1 was added to 200 $\mu$ℓ of the glycyrrhizin (400 $\mu$g/mℓ PBS) and heated in a boiling water bath for 30 minutes. After cooling down at ambient temperature, 400 $\mu$ℓ of 2N sodium hydroxide solution was added for neutralization (final concentration of glycyrrhizin was 100 $\mu$g/mℓ).

Following the same procedure as in the case of glycyrrhetic acid, a dilution series of the neutralized solution was prepared. The detection sensitivity of the series was examined by inhibition ELISA. Glycyrrhizin without acid hydrolysis was used as negative controls.

The results thus obtained are shown in Figs. 12, 13 and 14. The detection sensitivity of glycyrrhetic acid was about 10 pg/mℓ in case that the antibody generated by any hybridoma strain was used. Hence, it was indicated that the quantitative assay of glycyrrhetic acid was possible within the range of 10 pg/mℓ and 1 $\mu$g/mℓ. It was also indicated that glycyrrhizin can be assayed in the form of glycyrrhetic acid after it is acid hydrolyzed into glychrrhizin. In case that a biological body is a sample of this assay, a non-treated sample is measured to possibly assay glycyrrhetic acid, while the assay of glycyrrhizin can be done by reducing the measured values prior to the treatment from the measured values of the sample acid hydrolyzed.

By using the monoclonal antibody, the oleanan-type five-membered triterpene can be measured by enzyme immunoassay. The monoclonal antibody according to the present invention can be applied to imaging diagnostics.

The monoclonal antibody can be used to prepare a diagnostic kit, for example a kit composed of a microtiter plate, bovine serum albumin (BSA), glychyrrhizin-BSA conjugate, monoclonal antibody, washing buffers, goat anti-mouse IgG labeled with alkali phosphatase, p-nitrophenyl phosphate, and glycyrrhetic acid of known amounts to be used as controls for the assay.

## Claims

1.  A monoclonal antibody capable of recognizing pentacyclic oleanan-type triterpene.

2. An antibody according to claim 1 capable of competing with the GA2-20-6 (FERM BP-3389) antibody for binding to pentacyclic oleanan-type triterpene.

3. An antibody according to claim 1 or 2 capable of recognising a pentacyclic oleanan-type triterpene which is glycryrrhetic acid or a derivative thereof.

4. An antibody according to any one of the preceding claims which is of class IgG.

5. An antibody according to claim 4 which is an $IgG_1$ antibody.

6. An antibody according to any one of the preceding claims labeled with at least one of a radioactive label, a para-magnetic label, a fluorescent label and an enzyme label.

7. A hybridoma cell line capable of producing an antibody as claimed in any one of claims 1 to 6.

8. A method for preparing an antibody as claimed in any one of claims 1 to 5, which method comprises recovering the supernatant from a culture of a hybridoma cell line as claimed in claim 7.

9. A method according to claim 8 in which the hybridoma cell line is generated by fusing a myeloma cell with a spleen cell raised in the presence of a pentacyclic oleanan-type triterpene bonded to a carrier protein.

10. A method according to claim 9, wherein the carrier protein is key-hole limpet hemocyanin.

11. A method according to claim 9 or 10, wherein the spleen cell is a mouse spleen cell and the myeloma cell is a myeloma cell derived from mouse.

12. Use of an antibody as claimed in any one of claims 1 to 6 as a diagnostic reagent for measuring pentacyclic oleanan-type triterpene in a sample.

13. A use according to claim 12, wherein the pentacyclic oleanan-type triterpene is derived from a biological sample.

14. A diagnostic kit for measuring a pentacyclic oleanan-type triterpene comprising an antibody as claimed in any one of claims 1 to 6, the pentacyclic oleanan-type triterpene and a carrier protein.

15. A diagnostic kit according to claim 14, wherein the carrier protein is bovine serum albumin.

# FIG.1

# FIG.2

# FIG.3

mAb dilution
□ lot 0126 + lot 0218

# FIG.4

mAb dilution
□ lot 0126 + lot 0218

# FIG.5

□ lot 0218 + lot 0219

# FIG.6

□ GA + GL ◇ 1 △ 2 X 3 ▽ 4

# FIG.7

Inhibitor conc (ng/ml)
□ 5 + 6

# FIG.8

Inhibitor conc (ng/ml)
□ GA + GL ◇ 1 △ 2 X 3 ▽ 4

# FIG.9

# FIG.10

15

# FIG.11

# FIG.12

# FIG.13

# FIG.14